# EUROPÄISCHE PATENTSCHRIFT

(11) **EP 3 289 960 B1**
(45) Veröffentlichungstag und Bekanntmachung des Hinweises auf die Patenterteilung: **27.07.2022**
(21) Anmeldenummer: 17188245.9
(22) Anmeldetag: 29.08.2017
(51) Int. Cl.: A61B 1/00, G02B 6/04

(54) **BELEUCHTUNGSSYSTEM MIT HETEROGENER FASERANORDNUNG**
ILLUMINATION SYSTEM WITH HETEROGENEOUS FIBRE ASSEMBLY
SYSTÈME D'ÉCLAIRAGE POURVU D'ENSEMBLE FIBREUX HÉTÉROGÈNE

(30) Priorität: 31.08.2016 DE 102016216443
(43) Veröffentlichungstag der Anmeldung: 07.03.2018
(73) Patentinhaber: SCHOTT AG, 55122 Mainz (DE)
(72) Erfinder: SCHULTHEIS, Bernd, 55270 Schwabenheim (DE); RUSSERT, Hubertus, 65321 Heidenrod (DE); WERNER, Holger, 60596 Frankfurt (DE); TABOR, Kevin, Webster, MA 01570 (US)
(74) Vertreter: Schott Corporate IP

(56) Entgegenhaltungen:
- WO-A1-2016/066370
- DE-A1- 2 028 111
- DE-B4-102013 208 838
- US-A- 3 089 484
- US-A- 3 902 880
- US-A- 4 772 093
- US-A- 5 116 317
- US-A- 5 550 945
- US-A1- 2006 030 753
- US-A1- 2008 044 146
- US-A1- 2013 156 389
- US-A1- 2015 305 602
- US-A1- 2015 305 603
- US-B1- 6 487 326

## Beschreibung

Die Erfindung betrifft ein Beleuchtungssystem für ein Inspektionssystem, welches eine räumliche Faseranordnung mit einem optischen Element am distalen Ende der Faseranordnung umfasst, und dass die Faseranordnung in deren Querschnitt mindestens einen Bereich B aufweist, der gegenüber den Einzelfasern eines restlichen Bereichs Ader Faseranordnung Einzelfasern mit deutlich kleinerem aktiven Durchmesser aufweist und dass das optische Element am distalen Ende insbesondere zu diesem Bereich B korrespondiert. Das Beleuchtungssystem ist an eine Lichtquelle angeschlossen und/oder zumindest an einer solchen anschließbar und/oder einer zuordenbar. Die Erfindung umfasst ebenso die Kombination des Beleuchtungssystems in Kombination mit der Lichtquelle.

Derartige Inspektionssysteme sind aus dem Bereich der Endoskopie bekannt. Dabei wird eine üblicherweise schwer zugängliche Oberfläche, die inspiziert werden soll, mittels eines flexiblen oder starren Endoskops mit Licht, üblicherweise Weißlicht, beleuchtet. Über aufwendige Linsensysteme wird das zurückreflektierte Licht über separate Kanäle aus dem Endoskop ausgeleitet und in ein Kamerasystem projiziert. Derartige endoskopische Systeme kommen insbesondere im Bereich der Anlagen- oder Maschineninspektion z.B. zur Inspektion von Motoren oder Flugzeugtriebwerken einerseits und andererseits im Bereich der Medizintechnik, hier insbesondere zu minimal invasiven Eingriffen, zum Einsatz. Zudem müssen derartige Geräte im Medizinbereich zerlegbar und aufbereitbar sein. Als Aufbereitungsmethoden sind hier insbesondere Reinigungs- und/oder Desinfektionsverfahren mit stark basischen Lösungen und die Sterilisation mittels Autoklavieren bei Temperatur bis zu 135° C und typischen Dampfdrücken von etwa 3 bar zu nennen. Daher eignen sich insbesondere starre oder flexible Faseranordnungen aus Glas- oder Quarzglasfasern. Solche Endoskope sind in der Regel daher recht kostenintensiv und aufwendig herzustellen, insbesondere wenn hochaufgelöste Bilder der Oberfläche übertragen werden sollen.

Im Dentalbereich sind u.a. Geräte zur Aushärtung von Zahnfüllungen bekannt, bei denen intensives blaues Licht über Glasfaserstäbe zum zu behandelten Zahn geführt wird, wie dies z.B. in der WO 015073370 A1 und WO 2013/050589 A2 beschrieben wird.

Die EP 2891467 A1 beschreibt einen gebogenen Faserstab, der als Lichtleiter in einem Turbinengehäuse eines Dental-Bohrers eingebaut ist, wobei eine Bildinformation einer Kamera zuführbar ist. Dieser Lichtleiter weist am Auskoppelende des Faserstabs eine Linse auf. Auch hier besteht die Motivation darin, eine einfache Kontrolle der Zahnoberfläche zu ermöglichen.

Von der Anmelderin sind u.a. Ansätze bekannt, mit denen sich Faseranordnungen mit extrem kleinen Fasern zur Bildübertragung realisieren lassen. Derartige Bildleiter, auch Image-Guides genannt, bestehen aus einigen Zehntausend Einzelfasern und erlauben Auflösungen von typischerweise 3 bis 6 µm.

Aus der US 2008/0044146 A1 ist beispielsweise eine flexible Faseranordnung bekannt, die ein Faserbündel mit einem als Bildleiter ausgeführten Bereich und einen als Lichtleiter ausgeführten Bereich aufweist. Dem als Bildleiter ausgeführten Bereich des Faserbündels ist am distalen Ende ein optisches Linsensystem zugeordnet.

Die DE 10 2010 052479 A1 beschreibt eine Faseranordnung und ein Verfahren zur Herstellung einer solchen Anordnung, für einfache, vergleichsweise kostengünstige starre Bildleiter.

Aus der DE 10 2013 208 838 B4 der Anmelderin sind Faserstäbe bekannt, die insbesondere verbesserte optische Eigenschaften im blauen Spektralbereich aufweisen, was z.B. Anwendungen im Dentalbereich begünstigt.

Die DE 10 2013 219 039 A1 beschreibt Faserstäbe, bei denen ein optisches Element aus hochtransparentem LSR (Liquid Silicone Rubber) am Faserstabende angeordnet ist.

Der US 55 50 945 ist eine Anordnung mit den Merkmalen des Oberbegriffs des Anspruchs 1 zu entnehmen.

Für viele Anwendungen im Industrie- und/oder Medizinbereich und/oder der Analytik genügt ein einfaches, weniger aufgelöstes Bild der zu inspizierenden Oberfläche und/oder des zu inspizierenden Volumens, um insbesondere eine Oberflächenveränderung und/oder Positionierungskontrolle zu gewährleisten, ohne dass kostenintensive aufwendige optische Systeme, wie sie bei bisherigen endoskopischen Systemen zum Einsatz kommen, verwendet werden müssen.

Aufgabe der Erfindung ist es daher, ein Beleuchtungssystem, insbesondere eine Faseranordnung bereitzustellen, mit der eine einfache und kostengünstige Kamera- und/oder Sensorinspektionslösung realisiert werden kann.

Die Aufgabe der Erfindung wird durch ein Beleuchtungssystem mit den Merkmalen des Anspruchs 1 gelöst.

Mit einem derart heterogenen Faserstab lassen sich einerseits zu inspizierende Objekte beleuchten und andererseits das zurück reflektierte Bild für eine Überwachung einer Oberfläche hinsichtlich Veränderungen oder Auffälligkeiten oder einer Positionierungsüberwachung auswerten. Mit diesem Faserstab wird zudem ermöglicht, dass die Kamera vom zu inspizierendem Objekt beabstandet angeordnet werden kann, was insbesondere bei zu erwartenden Umgebungsbedingungen im Hinblick auf Feuchte und Temperatur von Vorteil ist. Für medizintechnische Anwendungen wird ermöglicht, dass die Faseranordnung für eine Aufbereitung Reinigungs- und Autoklavierprozessen unterzogen werden können. Durch diese heterogene Faseranordnung mit einem Bereich mit üblichem Einzelfaserdurchmesser für die Lichtleitung (Bereich A) und mindestens einem Bereich mit deutlich reduziertem Einzelfaserdurchmesser (Bereich B) für die Bildleitung kann zudem der Herstellungsaufwand gegenüber einem Ansatz mit reduziertem Einzelfaserdurchmesser über den gesamten Durchmesser des Faserstabs reduziert werden. Mit derartigen Faserstäben lassen sich insbesondere sogenannte Dental-Curing-Rods (deutsch: Dentalaushärtestäbe oder kurz Dentalstäbe) herstellen, die nach einer Behandlung des Patienten vom Aushärtegerät leicht demontiert und aufbereitet bzw. sterilisiert werden können. In Anwendungen im industriellen Bereich können damit einfache Durchführungen, z.B. in Maschineninnenräumen, Motoren, Triebwerken oder Heizungssysteme, mit geringem Montageaufwand realisiert werden. Ebenso ist es möglich, die Qualität von Verbindungen in der Verbindungstechnik zu inspizieren. In der Analytik und/oder der Gewässerüberwachung ist es ebenso möglich, die Anwesenheit von Körpern in der Flüssigkeit zu Quantifizieren und ggfls. zu charakterisieren.

In einer bevorzugten Ausführungsvariante der Erfindung ist vorgesehen, dass ein Einzelfaserkerndurchmesser B im Bereich B des Faserstabs einen Durchmesser von weniger als 25 µm, typischerweise von 10 bis 20 µm, bevorzugt weniger als 10 µm aufweist. Damit lassen sich zumindest einfach ausreichend gut aufgelöste Bilder der Objektoberfläche auf die Kamera projizieren. Üblicherweise liegt der Einzelfaserkerndurchmesser A im Bereich A des Faserstabs bei 40 bis 100 µm Durchmesser. Zur Herstellung kommen unterschiedliche Methoden in Frage, die aber alle das mehrfache Wiederziehen von Fasern umfassen. Je nach Herstellprozess kann der Bereich B als im Wesentlichen runder Bereich oder quadratischer bzw. rechteckiger oder hexagonaler Bereich ausgeführt sein, wobei quadratische oder rechteckige Formen hinsichtlich einer optimalen Flächennutzung im Hinblick auf die üblicherweise quadratische oder rechteckige aktive Fläche der Kamerachips vorteilhaft sind. Für sensorische Anwendungen können demgegenüber auch runde oder hexagonale Bereiche Vorteile bieten. Weiterhin kann auch vorgesehen sein, dass die Faseranordnung aus starren und flexiblen Bereichen A, B bestehen, d.h. eine starre Faseranordnung bildet dabei den Bereich A aus und besitzt mindestens einen Kanal, in dem ein flexibles Faserbündel als Bildleiter eigezogen ist, welches den Bereich B ausbildet.

In einer bevorzugten Ausführungsform für den Faserstab ist vorgesehen, dass der Bereich C derart bemessen ist, dass Streulichtübertritte zwischen den Bereichen A und B um einen Faktor ≥ 20, gedämpft sind.

Die Erfinder haben erkannt, dass durch die Ausführung der Faseranordnung als Faserstab, bei dem die Einzelfasern zumindest in Teilbereichen ihrer Außenumfangsflächen miteinander verschmolzen und/oder miteinander versintert und/oder miteinander verklebt sind, die Wahrscheinlichkeit des Übersprechens von geleitetem Licht von einer Einzelfaser in den nächsten steigt. Somit ist es auch möglich, dass im Betriebszustand im Bereich A geleitetes Licht und/oder vom Objekt am distalen Ende reflektiertes und im Bereich A zurückreflektiertes Licht in den für die Leitung der Bildinformation zuständigen Bereich B überspricht. Solches übersprechende Licht kann zu bedeutenden Verschlechterungen der Bildqualität führen und Untersuchungen unmöglich machen. Dies ist ein prinzipieller Nachteil des dem vereinfachten Herstellungsverfahren geschuldeten Faserstabs. Wird der Bereich C sozusagen als Zwischenbereich zwischen den Bereichen A und B wie beschrieben ausgeführt sorgt dieser Bereich C auf überraschend einfache Weise dafür, dass solche Übersprechungsphänomene zumindest deutlich reduziert werden, wodurch die durch das erfindungsgemäße Beleuchtungssystem erzielbare Bildqualität verbessert wird.

Ist der Bereich C opak ausgeführt, genügt bereits eine wenige µm dicke Wandstärke C. Bei einer im Wesentlichen transparenten Abtrennung muss diese im Allgemeinen dicker ausgeführt sein. Als vorteilhaft haben sich hier insbesondere eingefärbte Abtrennungen erwiesen, die einerseits als opake Trennung oder als Farbfilter wirken. Zudem können opake, streulichtabsorbierende Faserelemente zwischen den Einzelfasern den Kontrast verstärken.

Hinsichtlich der Robustheit und insbesondere im Hinblick auf die chemische Beständigkeit als auch im Hinblick auf die bei Aufbereitungszyklen mittels Autoklavieren auftretenden Temperaturen ist es besonders vorteilhaft, wenn die Einzelfasern der Faseranordnung aus Glasfasern, bestehend aus einem Kern- und einem Mantelglas bestehen. Glassysteme mit hohen Transmissionswerten im sichtbaren, insbesondere im blauen Spektralbereich unterhalb 520 nm und/ oder im nahen IR-Bereich bis 1000 nm bieten zudem den Vorteil, dass einerseits blaues Licht mit hoher Intensität für Anwendungen im Bereich Dentalmedizin zur Aushärtung von Zahnfüllungen effizient übertragen werden kann. Hier kommt es insbesondere auf eine hohe Übertragungseffizienz im Bereich insbesondere der Wellenlängenbereich von 380 nm bis 510 nm mit Schwerpunkten hinsichtlich einer optimalen Absorption des Harzmaterials der Zahnfüllung bei 380 nm bis 390 nm, 460 nm bis 470 nm und/ oder 490 nm bis 520 nm an. Zudem können Lichtverluste im Gesamtsystem infolge insbesondere der Abschattung im Zentrum zumindest teilweise kompensiert werden. Andererseits ermöglicht auch eine hohe Transmission im nahen IR-Bereich Anwendungen, bei denen z.B. ein Bild mit den üblichen IR-Detektor im Bereich von 850 nm bis 950 nm aufgenommen werden kann. Dies ist für bestimmte Anwendungen im Bereich Medizintechnik als auch im industriellen Umfeld von Vorteil.

Besteht das Kern- und Mantelglas, sowie die Abtrennung im Bereich C aus einer Blei- bzw. Schwermetallfreien Glaszusammensetzung, können zudem noch zukünftige RoHS-Anforderungen erfüllt werden. Ebenso ist eine Anordnung mit Quarzfasern denkbar, womit insbesondere Anwendungen im UV und/ oder IR-Bereich adressiert werden können.

Beispiele für derartige Gläser für das Kernglas sind Systeme aus dem Bereich der bleifreien Silikat-Zinn-Gläser die folgende Komponenten beinhalten (angegeben in Gew.% auf Oxidbasis):

| | von | bis |
|---|---|---|
| B₂O₃ | 0 | 24 |
| SiO₂ | 23 | 62,1 |
| Al₂O₃ | 0 | 10 |
| Li₂O | 0 | 10 |
| Na₂O | 0 | 18,5 |
| K₂O | 0 | 25,7 |
| BaO | 0 | 57,8 |
| ZnO | 0 | 40 |
| La₂O₃ | 0 | 25 |
| ZrO₂ | 0 | 10 |
| HfO₂ | 0 | 14,2 |
| SnO₂ | >0 | 2 |
| MgO | 0 | 8 |
| CaO | 0 | 8 |
| SrO | 0 | 24,4 |
| Ta₂O₅ | 0 | 22 |
| Y₂O₃ | 0 | 11,9 |
| Rb₂O | 0 | 15 |
| Cs₂O | 0 | 21 |
| GeO₂ | 0 | 7,5 |
| F | 0 | 2 |
| Σ R₂O | 5 | 20 |
| Σ MgO, CaO, SrO, ZnO | 20 | 42 |

Das Mantelglas bzw. die Abtrennung im Bereich C ist vorzugsweise aus einer der folgenden Gruppe 1 bis 4 aufgebaut, welches jeweils nachfolgende Komponenten beinhalten (angegeben in Gew.% auf Oxidbasis), wobei die Abtrennung im Bereich C ein z.B. schwarz oder braun eingefärbtes Mantelglas bzw. Hüllglas mit dieser Zusammensetzung sein kann.

| | **Gruppe 1** | **Gruppe 2** | **Gruppe 3** | **Gruppe 4** |
|---|---|---|---|---|
| SiO₂ | 70 - 78 | 63 - 75 | 75 - 85 | 62 - 70 |
| Al₂O₃ | 5 - 10 | 1 - 7 | 1 - 5 | 1 - 10 |
| B₂O₃ | 5 - 14 | 0 - 3 | 10 - 14 | > 15 |
| Li₂O | 0 - 2 | 0 - 1 | 0 - 3 | 0 - 2 |
| Na₂O | 0 - 10 | 8 - 20 | 2 - 8 | 0 - 10 |
| K₂O | 0 - 10 | 0 - 6 | 0 - 1 | 0 - 10 |
| MgO | 0 - 1 | 0 - 5 | 0 | 0 - 5 |
| CaO | 0 - 2 | 1 - 9 | 0 | 0 - 5 |
| SrO | 0 - 1 | 0 | 0 | 0 - 5 |
| BaO | 0 - 4 | 0 - 5 | 0 | 0 - 5 |
| F | 0 - 1 | 0 - 1 | 0 | 0 - 1 |
| Cl | 0 - 1 | 0 - 1 | 0 | 0 - 1 |
| Fe₂O₃ | 0 - 2 | 0 - 2 | 0 - 2 | 0 - 2 |

Eine entsprechende Faseranordnung kann auch aus Quarzfasern bestehen, bei denen eine Quarzfaser ein sogenanntes Cladding aus organischen Materialien aufweist. Derartige Faseranordnungen bieten insbesondere Vorteile bei der Übertragung von Licht im UV- oder IR-Bereich.

Es kann auch vorteilhaft sein, wenn der Bereich A und der Bereich B aus unterschiedlich transmittierenden Lichtleitsystemen besteht, die eine unterschiedliche Glaszusammensetzung aufweisen können. So können die Einzelfasern für den Bereich A auf den zur Beleuchtung optimierten Lichtwellenbereich, z.B. blaues Licht oder UV-Licht, ausgewählt sein und die Einzelfasern des Bereichs B aus Fasern bestehen, die auf einen anderen Bereich des Spektrums, z.B. für den IR-Bereich, optimiert sind.

Für Anwendungen im Bereich niedrigerer Temperaturen können aber auch alternativ die Einzelfasern aus optisch leitfähigen Kunststofffasern, z.B. aus Polycarbonat oder PMMA bestehen, was hinsichtlich der Herstellkosten Vorteile bieten kann.

In einer bevorzugten Ausführungsvariante kann vorgesehen sein, dass der Faserstab mindestens eine Biegung und/ oder eine Verjüngung zwischen dem proximalen und dem distalen Ende des Faserstabs aufweist oder als räumlich verformter Faserstab ausgeführt ist, der entlang seiner Längsachse einen unterschiedlichen Querschnitt aufweisen kann. Derartige Geometrien werden insbesondere im Dentalbereich für Aushärtegeräte, wie sie eingangs erwähnt wurden, oder für Lichtleiter in Turbinengehäusen verwendet. Die Biegung ermöglicht z.B., dass Backenzähne gut im Mundraum erreicht werden können. Mit einer Verjüngung des Faserstabs kann das Licht auf kleinere Bereiche konzentriert und damit die Intensität erhöht werden.

Zur Fokussierung des reflektierten Lichtes von der Objektoberfläche in den Bereich B des Faserstabs ist das optische Element am distalen Ende als Gradientenindex-Linse, Kugellinse, asphärische Linse, Fresnel-Linse oder als LSR-Linse ausgeführt. Gradientenindex-Linsen können als sehr kompakte zylindrische Baugruppe im Faserstabende montiert werden und erlauben qualitativ eine ausreichend gute Abbildung. Kugellinsen sind demgegenüber preiswert in der Herstellung. Qualitativ hochwertig sind asphärisch geschliffene Linsen, welche insbesondere aus Pb- bzw. schwermetallfreien Gläsern hergestellt werden können. Denkbar sind auch sogenannte LSR-Linsen, die aus einem hochtransparenten Silikon bestehen, welche zuvor mittels Spritzguss hergestellt und aufgeklebt oder direkt an das distale Ende des Faserstabs angespritzt werden kann.

Besonders vorteilhaft ist dabei eine Einbettung des optischen Elements in eine Abdeckscheibe, vorzugsweise aus Glas hergestellt, die mit einem optischen Kleber auf das distale Ende der Faseranordnung bzw. des Faserstabs befestigt ist oder in einer Bohrung, hier als Sackbohrung ausgeführt, des Faserstabs eingeklebt ist. Der optische Kleber mit angepasstem Brechungsindex zu dem des Kernmaterials der Einzelfasern ermöglicht es, dass Reflexionsverluste weitgehend reduziert werden können. Zudem kann eine mechanische Bearbeitung (Schleifen und Polieren) der Abdeckscheibe mit dem optischen Element separat von der Bearbeitung des Faserstabs erfolgen. Diese wird lediglich nach erfolgtem Planschliff und Politur auf das Faserstabende geklebt. Durch geeignete Wahl des Klebers kann auch erreicht werden, dass Streulicht zwischen den lichtleitenden Bereichen und den Bildleitenden Bereichen reduziert werden kann. Dazu eignen sich besonders eingefärbte Kleber, die an der Mantelfläche des optischen Elements aufgebracht werden. Zur Streulichtunterdrückung sind auch separate zylindrische Hülsen aus Metall oder opaken Kunststoff zwischen optischen Element und Abdeckscheibe denkbar.

Für eine effektive Bildauskopplung hat sich als vorteilhaft herausgestellt, wenn das optische Koppelelement einen Strahlteiler aufweist, in den das Licht der Lichtquelle über ein Fokusierelement in Form einer Linsenanordnung und/ oder eines Reflektors einkoppelbar ist und das derart kollimierte Licht in das proximale Ende des Faserstabs einkoppelbar ist, und dass das Bild am proximalen Ende des Faserstabs aus dem Bereich des Faserstabs mit kleinerem Einzelfaserkerndurchmesser auf eine aktive Fläche der Kamera mittels eines Linsensystems projizierbar ist.

Um eine Abbildung der Objektoberfläche zu verbessern und dabei ein Überstrahlen der Kamera vor allem zu verhindern, kann in einer bevorzugten Ausführungsvariante vorgesehen sein, dass die Lichtquelle zur Beleuchtung eines Objektes mit seiner Objektoberfläche einen LED-Typ A und zusätzlich einen LED-Typ B mit zum LED-Typ A unterschiedlicher Lichtfrequenz und das optische Koppelelement an dessen Projektionsausgangs zur Kamera einen optischen Filter aufweist, der ein Durchlassverhalten für die Lichtfrequenz des LED-Typs B aufweist. Hiermit können insbesondere störende Reflexionen am Strahlteiler, verursacht vom Licht der Hauptlichtquelle mit dem LED-Typ A, gefiltert werden. Bei der Auswahl der LED-Typen A, B ist insbesondere darauf zu achten, dass die emittierten Spektralbereiche der LED-Typen A und B sich möglichst nicht überlappen und der Filter hinsichtlich seines Durchlassverhaltens als schmalbandiger Farbfilter mit hoher Trenngüte ausgelegt ist.

Dabei ist z.B. für eine Anwendung in einem Dental-Aushärtegerät vorgesehen, dass die Lichtquelle einen LED-Typ A mit einem Strahlungsspektrum von ≤ 520 nm und einen LED-Typ B mit einem Strahlungsspektrum im grünen (etwa bei 550 nm), gelben (etwa bei 580 nm bis 600 nm) oder roten sichtbaren Spektralbereich (etwa zwischen 630 nm und 780 nm) oder im IR-Spektralbereich aufweist. Hier sei angemerkt, dass für Anwendungen im Bereich der IR-Detektion bzw. -überwachung auch der LED-Typ A im NIR-Bereich, z.B. zwischen 800 nm und 1000 nm strahlt und der LED-Typ B z.B. im roten sichtbaren Spektralbereich zwischen 630 nm und 780 nm emittiert. Grundsätzlich sind, je nach Anwendung, auch andere LED-Kombinationen denkbar.

Im Hinblick auf eine Anwendung, bei der es insbesondere auf den Kontakt zwischen Faserstab und der gleichzeitig zu beobachteten Objektoberfläche ankommt, kann vorteilhaft vorgesehen sein, dass der Faserstab mit seinen Bereichen A, B und C und dem optischen Element an seinem distalen Ende einen optischen Abstandshalter aufweist, wobei der optische Abstandshalter als Hülse oder als transparenter Körper, bestehend aus Glas, Quarz oder Kunststoff, insbesondere als Lichtleitelement ausgeführt sein kann. Eine derartige Anforderung ergibt sich beispielsweise, wenn im Dentalbereich der Faserstab zur Aushärtung der Zahnfüllung in Kontakt mit dem zu behandelnden Zahn gebracht wird und gleichzeitig das Bild der gesamten Zahnoberfläche erfaßt werden soll. Durch den optischen Abstandhalter kann dies gewährleistet werden.

Die Erfindung sieht als bevorzugte Verwendung des Beleuchtungssystems, wie es zuvor beschrieben wurde, den Einsatz in einem Inspektionssystem zur Überwachung von Anlagen oder Maschinen vor. Damit können einfache bildgebende Überwachungsinstrumente insbesondere für temperatur-, feuchte- und/ oder schmutzbelastende Umgebungen realisiert werden, bei denen eine Auflösung im Bereich von typisch 5 µm bis 10 µm Pixelgröße ausreichend ist. Ein weiteres bevorzugtes Anwendungsfeld sieht den Einsatz für ein endoskopisches Instrument im Bereich der Medizintechnik oder für ein Aushärtegerät für Zahnfüllungen im Bereich Dentaltechnik oder für eine otoskopische Anwendung vor. Hier ergibt sich insbesondere der Vorteil, dass derartige Faseranordnungen bzw. Faserstäbe den empfindlichen Kamerateil schützen und zur medizintechnischen Aufbereitung leicht demontiert und später wieder montiert werden können. Zudem ergibt sich damit auch eine galvanische Trennung zwischen elektrisch führenden Teilen und dem Gewebe bzw. der zu behandelnden Oberfläche. Ebenfalls wird dadurch auch die Austauschbarkeit im Falle eines Defekts erleichtert.

Eine weitere Anwendung im medizintechnischen Bereich bezieht sich auf eine optische Biopsie-Anwendung, bei der ein Fluoreszenzbild von der zu untersuchenden Gewebeoberfläche analysiert werden kann. Dabei wird die Gewebeoberfläche mit Licht einer bestimmten Wellenlänge, je nach Anwendung kann dies im blauen bzw. nahen UV-Bereich oder aber auch im infraroten Bereich des Spektrums liegen, bestrahlt und dabei eine Fluoreszenz angeregt. Dieses Fluoreszenzbild kann dann mittels der Kamera oder eines Sensors ausgewertet werden. Damit lassen sich beispielsweise Gewebeveränderungen frühzeitig detektieren. Weitere vorteilhafte Verwendungen ergeben sich auch bei der industriellen Kleberaushärtung mittels UV-Licht. Dabei kann z.B. eine Positionierungskontrolle realisiert werden.

Ebenso vorteilhaft kann ein derartiges System auch beim Laserschweißen an schwer zugänglichen Werkstückoberflächen eingesetzt werden. Damit kann eine zusätzliche Inspektionsfunktion realisiert werden. Allgemein sind jegliche Anwendungen in der Verbindungstechnik mit dem erfindungsgemäßen Beleuchtungssystem möglich, insbesondere auch die Inspektion von Nietverbindungen und/oder allgemein in der Klebetechnik.

Ein weiteres vorteilhaftes Anwendungsgebiet ist die Analytik, hierbei insbesondere die Überwachung und/oder Prüfung von flüssigen Stoffen. Mit der erfindungsgemäßen Beleuchtungseinrichtung ist es z.B. möglich, die Anwesenheit von Körpern, z.B. Schwebstoffen, in Flüssigkeiten und/oder Gasen zu identifizieren und/oder zu quantifizieren. Insbesondere über optische Methoden erscheint sogar die Charakterisierung und/oder Identifikation dieser Körper möglich. Entsprechend ist ein bevorzugtes Anwendungsgebiet die Gewässerüberwachung, einschließlich Prozessschritte der Trinkwasseraufbereitung.

Die Erfindung wird im Folgenden anhand eines in den Figuren dargestellten Ausführungsbeispiels näher erläutert. Es zeigen:
Figur 1: schematisch ein Beleuchtungssystem mit einem Faserstab,
Figur 2: einen Querschnitt durch den Faserstab,
Figur 3a bis 3c: verschiedene Ausführungsbeispiele für ein optisches Element am Auskoppelende des Faserstabs,
Figur 4: eine spezielle Ausführungsform des Auskoppelendes des Faserstabs und
Figur 5a und 5b: schematisch das Größenverhältnis zwischen aktiver Fläche der Kamera und dem für die Bildleitung vorgesehen Bereich des Faserstabs.

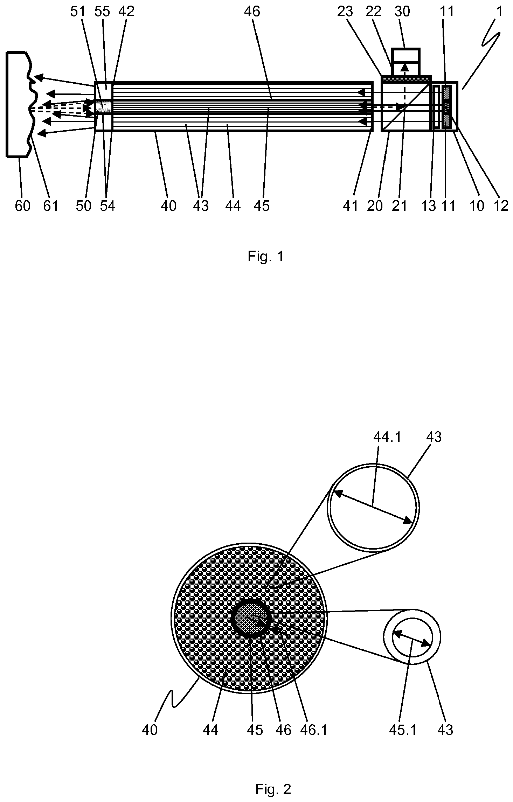

Figur 1 zeigt schematisch den Aufbau eines Beleuchtungssystems 1 gemäß der Erfindung. Beispielhaft ist hier eine starre Faseranordnung in Form eines Faserstabs 40 dargestellt.

Im gezeigten Beispiel besteht das Beleuchtungssystem 1 aus einer Lichtquelle 10, welche als LED-Lichtquelle mit vorwiegend einem LED-Typ A 11 ausgestattet ist, welcher Licht in einem bestimmten Spektralbereich aussendet. Zusätzlich kann optional in der Lichtquelle 10 noch ein zweiter LED-Typ B 12 angeordnet sein, der Licht in einem zweiten Spektralbereich aussendet.

Für ein Aushärtegerät für Zahnfüllungen sind beispielsweise die LEDs vom LED-Typ A 11 als blau leuchtende LEDs ausgeführt. Die Wellenlänge des blauen Lichts liegt üblicherweise zwischen 390 nm und 520 nm und ist dabei insbesondere auf die Reaktionskinetik des Zahnfüllmaterials abgestimmt. Die Wellenlänge der LEDs vom LED-Typ B 12 kann beispielsweise im grünen oder roten sichtbaren Spektralbereich liegen und hat idealerweise keinen Überlapp zum Spektralbereich des LED-Typs A 11.

Für andere Anwendungen können die LEDs vom LED-Typ A 11 auch als Weißlicht-LEDs ausgeführt sein, wobei die LEDs vom LED-Typ B 12 in diesem Fall auch im NIR-Bereich, beispielsweise zwischen 800 nm und 1000 nm, abstrahlen können.

Das Licht der Lichtquelle 10 wird über ein Fokusierelement 13 in ein optisches Koppelelement 20 in das proximale Ende 41 des Faserstabs 40 eingekoppelt. Das Fokusierelement 13 kann dabei als Linsenanordnung und/ oder Reflektor ausgeführt sein. Ebenso denkbar sind auch Lichtleitstäbe, die zur Lichtmischung dienen und damit eine homogenere Lichteinkopplung ermöglichen. Das optische Koppelelement 20 weist einen Strahlteiler 21 auf, mit dem vom Faserstab 40 zurückreflektiertes Licht seitlich zu einer Kamera 30 ausgekoppelt werden kann.

Der Faserstab 40 mit einem Durchmesser von typischerweise 6 bis 12 mm besteht aus einer Faseranordnung mit etlichen tausend Einzelfasern 43, die zueinander im Wesentlichen parallel ausgerichtet sind, wobei erfindungsgemäß der Faserstab einen Bereich A 44 mit Einzelfasern 43 aufweist, die insbesondere zur Lichtleitung dienen, und einen Einzelfaserkerndurchmesser A 44.1 aufweisen, der üblicherweise im Bereich von 40 bis 100 µm liegt. Im Zentrum des Faserstabs 40 weist der Faserstab 40 einen Bereich B 45 auf, bei dem die Einzelfasern 43 einen Einzelfaserkerndurchmesser B 45.1, abhängig vom Faserziehverfahren, im Bereich von 10 bis 20 µm, vorzugsweise von weniger als 10 µm aufweisen. Dieser Bereich B 45 dient als Bildleiter und ist durch einen Bereich C 46 vom Bereich A 44 getrennt.

Am distalen Ende 42 des Faserstabs 40 ist ein optisches Element 50 angebracht, welches das zurückreflektierte Licht einer Objektoberfläche 61 eines zu inspizierenden Objekts 60 fokussieren und in den Bereich B 45 des Faserstabs 40 einkoppelt. Im gezeigten Beispiel ist das optische Element als zylinderförmige Gradientenindex-Linse 51 (auch als GRIN-Linse bekannt) ausgeführt, welche in einer Abdeckscheibe 55 aus Glas oder transparentem Kunststoff zum Bereich B 45 des Faserstabs 40 zentriert positioniert ist. Die Gradientenindex-Linse 51 und die Abdeckscheibe 55 sind mit einem optischen Kleber 54, üblicherweise ein hochtransparenter und temperaturbeständiger 2-Komponenten-Epoxidharz-Kleber, mit dem Faserstab 40 verklebt.

Die mittels des optischen Elements 50 im Bereich B 45 des Faserstabs 40 geführte Bildinformation wird am proximalen Ende 41 des Faserstabs über dem Strahlteiler 21 zur Kamera 30 mittels eines Linsensystems 22 projiziert. Um Reflektionen des in der Regel intensiven Lichts der LEDs vom LED-Typ A 11 zu unterdrücken, ist vor der Kamera 30 ein Filter 23 vorgesehen, der lediglich das Licht der LEDs vom LED-Typ B 12 passieren lässt.

Figur 2 zeigt den Querschnitt des Faserstabs 40 mit den Bereichen A, B und C 44, 45, 46. Schematisch, nicht maßstabsgetreu, sind die unterschiedlichen Einzelfaserkerndurchmesser A, B 44.1, 45.1 der Einzelfasern 43 dargestellt, welche auch als aktive Faserfläche bezeichnet wird. Dabei kann vorgesehen sein, dass das Verhältnis zwischen dem Einzelfaserkerndurchmesser B 45.1 zum Durchmesser der Einzelfasern 43 im Bereich B 45 kleiner ist als das Verhältnis zwischen dem Einzelfaserkerndurchmesser A 44.1 zum Durchmesser der Einzelfasern 43 im Bereich A 44. Dies verhindert während des Herstellprozesses, dass die Manteldicke der Einzelfasern 43 zu dünn wird, und dadurch Kontrastverluste oder Beeinträchtigungen der Bildinformationsübertragung auftreten. Beabstandet ist der Bereich A 44 vom Bereich B 45 durch einen Bereich C 46, der eine bestimmte Wandstärke 46.1 besitzt, die so bemessen ist, dass möglichst kein Streulichtaustausch zwischen den Bereichen A und B 44, 45 auftritt. Dazu kann vorgesehen sein, dass der Bereich C 46 aus einem opaken, transluzenten und/ oder eingefärbten, als Filterschicht wirkendem Material besteht.

Diese wie ebenso alle übrigen Zeichnungen sind rein schematisch. Ihnen sind keine realen Größenverhältnisse zu entnehmen. Insbesondere bei der Verwendung von Glasmaterialien als Fasermaterial des Faserstabs verschmelzen und/oder versintern die Fasern beim Ausziehen vorteilhaft an ihren Außenumfangsflächen. Der Querschnitt der Einzelfasern nähert sich üblicherweise einer hexagonalen Form an.

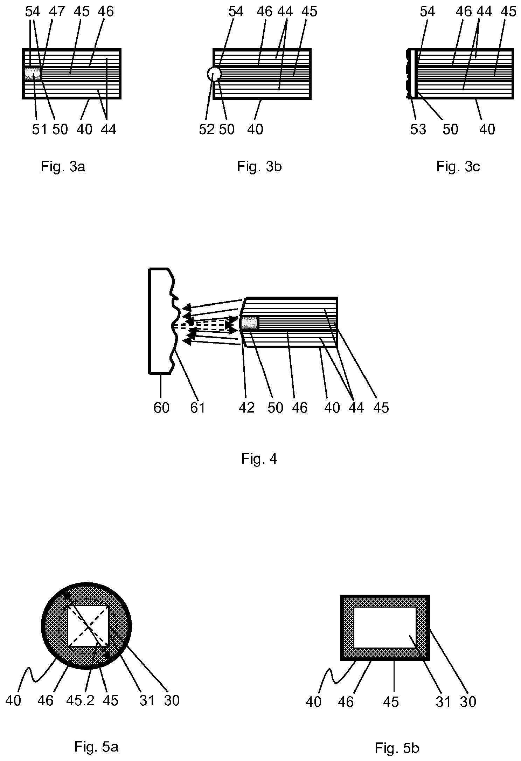

In den Figuren 3a bis 3c sind verschiedenen Ausführungsbeispiele für das optische Element 50 gezeigt. Die Aufgabe des optischen Elements 50 ist es, das von der Objektoberfläche 61 des zu inspizierenden Objektes 60 zu bündeln und das Bild in den Bereich B 45 des Faserstabs 40 einzukoppeln. Daher kommen hier Sammellinsen zum Einsatz.

In Figur 3a ist die Gradientenindex-Linse 51 als optisches Element 50, wie sie bereits in Figur 1 gezeigt ist, direkt in das Ende des Faserstabs 40 integriert. Dazu ist im Bereich B 45 eine Bohrung 47 vorgesehen, in die die Gradientenindex-Linse 51 mittels des optischen Klebers 54 eingeklebt ist.

Figur 3b zeigt als optisches Element 50 eine Kugellinse 52, die in eine entsprechende kugelförmige Vertiefung des Faserstabs 40 im Bereich des Bereichs B 45 mit dem optischen Kleber eingeklebt ist.

In Figur 3c ist das optische Element 50 als Fresnel-Linse 53 ausgeführt, welche auf deren Oberfläche unterschiedliche ringförmige Strukturen zur Fokussierung des von der Objektoberfläche 61 zurück reflektierten Lichts und aber auch zur Fokussierung des Lichts auf die zu inspizierende Objektoberfläche 61 aufweisen kann. Idealerweise ist diese Fresnel-Linse 53 als gepresste Glas- oder Kunststoffscheibe ausgeführt und ist mit dem optischen Kleber 54 auf den Faserstab 40 geklebt.

In einer bevorzugten Ausführungsvariante des Faserstabs 40 kann, wie dies Figur 4 zeigt, vorgesehen sein, dass das distale Ende 42 des Faserstabs 40 im Bereich A 44 leicht schräg oder "ballig" endenbearbeitet ist. Damit kann erreicht werden, dass das Licht, welches über die Fasern im Bereich A 44 geführt wird, beim Austritt aus dem Faserstab 40 zur Mittelachse des Faserstab 40 gebeugt wird, was einen Fokussierungs- und Homogenisierungseffekt mit sich bringt. Die Lichtabschattung durch den Bereich B 45 auf der Objektoberfläche 61 kann damit zumindest teilweise kompensiert werden.

Figur 5a zeigt zur Auslegung des Durchmessers B 45.2 des Bereichs B 45 zur aktiven Fläche 31 der Kamera 30 oder des Sensors, dass der Durchmesser B 45.2 des Bereichs B 45 im Minimum der Diagonalen des üblicherweise quadratischen oder rechteckigen Kamerachips entspricht zuzüglich einer Toleranzreserve, welche für die Positionierung erforderlich ist. Bei einer aktiven Fläche 31 von 1 × 1 mm ergibt sich ein minimaler Durchmesser B 45.2 des Bereichs B 45 von etwa 2,0 bis 2,5 mm, wenn als Positionierungstoleranz ein Maß von 0,3 bis 0,5 mm angenommen wird.

Figur 5b zeigt die Situation, wie in Figur 5a dargestellt, bei einem rechteckigen Bereich B 45 des Faserstabs 40. Die Abmessung des Bereichs B 45 ist hier rundumlaufend etwa 0,3 bis 0,5 mm größer ausgelegt als die aktive Fläche 31 der Kamera 30 oder des Sensors.

Hinsichtlich der Vermeidung von störendem Streulicht zwischen dem Bereich A 44 und dem Bereich B 45 ist der Bereich C 46 vorgesehen, wofür als Streulicht absorbierende Abtrennung ein schwarz eingefärbtes Hüllglas zwischen den Bereichen A und B 44, 45 verwendet wird. Weiterhin denkbar ist das Einbringen von opak eingefärbten Faserelementen zwischen den Einzelfasern 43. Damit kann eine Kontrastverstärkung infolge der Vermeidung von Streulicht zwischen den Einzelfasern 43 erzielt werden.

Grundsätzlich sind für bestimmte Anwendungen auch zwei und mehrere Bereiche B 45 innerhalb der Faseranordnung bzw. des Faserstabs 40 denkbar, z.B. ein erster Bereich B 45 für eine Bildübertragung und ein weiterer Bereich B 45, der räumlich vom ersten Bereich B 45 getrennt angeordnet ist, für die Übertragung eines Sensorsignals.

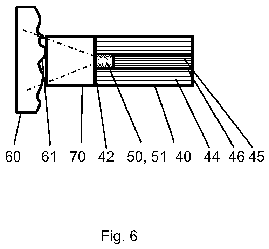

Figur 6 zeigt eine Anordnung, bei der der Faserstab 40 mit seinen Bereichen A 44, B 45 und C 46 und dem optischen Element 50, welches im gezeigten Beispiel als Gradientenindex-Linse (GRIN-Linse) 51 ausgeführt ist, an seinem distalen Ende 42 einen optischen Abstandshalter 70 aufweist, wobei der optische Abstandshalter als Hülse oder als transparenter Körper, bestehend aus Glas, Quarz oder Kunststoff (z.B. aus PC, PMMA), insbesondere als Lichtleitelement ausgeführt sein kann. Damit kann der Faserstab 40 in Kontakt mit der Objektoberfläche 61 des Objektes 60 gebracht werden, wobei gleichzeitig ein Abbild der vom Querschnitt des Abstandshalters überdeckten Objektoberfläche 61 aufgenommen werden kann.

Anstelle der Kamera 30 kann auch ein Sensor, z.B. ein IR- oder UV-Sensor, angeordnet sein. Ein derartiger Sensor kann beispielsweise verschiedene Zonen aufweisen, wobei mittels der Faseranordnung eine räumliche Signal-Auswertung ermöglicht wird. Insbesondere bei Verwendung der zuvor beschriebenen Glaszusammensetzungen kann im nahen UV-Bereich (typ. 350 nm bis 400 nm) eine UV-Applikation ermöglicht werden.

Die erfindungsgemäße Beleuchtungseinrichtung hat den Vorteil, dass sie mit reduziertem Herstellungsaufwand herstellbar ist und verglichen mit den endoskopischen Lösungen aus dem Stand der Technik zwar eine verminderte Bildqualität bereitstellt, die Bildqualität aber angesichts des geringen Herstellungsaufwand so gut ist, dass vielfältige Anwendungen auch in hohen Stückzahlen ermöglicht werden.

**Bezugszeichenliste:**

| | |
|---|---|
| 1 | Beleuchtungssystem |
| 10 | Lichtquelle |
| 11 | LED Typ A |
| 12 | LED Typ B |
| 13 | Fokusierelement |
| 20 | optisches Koppelelement |
| 21 | Strahlteiler |
| 22 | Linsensystem |
| 23 | Filter |
| 30 | Kamera |
| 31 | Aktive Fläche |
| 40 | Faserstab |
| 41 | proximales Ende |
| 42 | distales Ende |
| 43 | Einzelfaser |
| 44 | Bereich A |
| 44.1 | Einzelfaserkerndurchmesser A |
| 45 | Bereich B |
| 45.1 | Einzelfaserkerndurchmesser B |
| 45.2 | Durchmesser Bereich B |
| 46 | Bereich C |
| 46.1 | Wandstärke C |
| 47 | Bohrung |
| 50 | optisches Element |
| 51 | Gradientenindex-Linse (GRIN-Linse) |
| 52 | Kugellinse |
| 53 | Fresnel-Linse |
| 54 | Kleber |
| 55 | Abdeckscheibe |
| 60 | Objekt |
| 61 | Objektoberfläche |
| 70 | Optischer Abstandshalter |

## Patentansprüche

1. Beleuchtungssystem (1) für ein Inspektionssystem, welches an eine Lichtquelle (10) angeschlossen ist und welches zumindest eine aus einer Vielzahl von Einzelfasern gebildete räumliche Faseranordnung mit einem optischen Element (50) am distalen Ende (42) der Faseranordnung umfasst, wobei die Faseranordnung in deren Querschnitt mindestens einen Bereich B (45) aufweist, der gegenüber den Einzelfasern (43) eines restlichen Bereichs A (44) der Faseranordnung Einzelfasern (43) mit deutlich kleinerem aktiven Durchmesser aufweist, und wobei das optische Element (50) am distalen Ende (42) zumindest diesem Bereich B (45) zugeordnet ist,
wobei die Faseranordnung als starrer Faserstab (40) ausgebildet ist, wobei zumindest die Einzelfasern im Bereich A (44) und die Einzelfasern im Bereich B (45) zumindest in Teilbereichen ihrer Außenumfangsflächen miteinander verschmolzen oder zusammengesintert oder miteinander verklebt sind,
**dadurch gekennzeichnet, dass**
zwischen dem Bereich A (44) und dem Bereich B (45) ein Bereich C (46) vorgesehen ist, welcher im Betriebszustand Streulichtübertritte zwischen den Bereichen A und B (44, 45) des Faserstabs (40) unterdrückt,
wobei der Bereich C (46) als opake oder transluzente oder weitgehend transparente oder eingefärbte Abtrennung ausgeführt ist,
deren Wandstärke C (46.1), abhängig vom Transmissionsgrad der Abtrennung für den Wellenlängenbereich der Lichtquelle (10) derart bemessen ist, dass Streulichtübertritte zwischen den Bereichen A und B (44, 45) um mindestens einen Faktor 10 gedämpft sind.

2. Beleuchtungssystem (1) nach Anspruch 1,
**dadurch gekennzeichnet, dass**
am proximalen Ende (41) des Faserstabs (40) ein optisches Koppelelement (20) vorgesehen ist, mit dem im Betriebszustand Licht aus der Lichtquelle (10) in den Faserstab (40) einkoppelbar und ein Bild am proximalen Ende (41) des Faserstabs (40) aus dem Bereich B (45) in eine Kamera (30) oder in einen Sensor einkoppelbar ist.

3. Beleuchtungssystem (1) nach mindestens einem der vorhergehenden Ansprüche
**dadurch gekennzeichnet, dass**
ein Einzelfaserkerndurchmesser B (45.1) im Bereich B (45) des Faserstabs (40) einen Durchmesser von weniger als 25 µm aufweist.

4. Beleuchtungssystem (1) nach mindestens einem der vorhergehenden Ansprüche,
**dadurch gekennzeichnet, dass**
zwischen den Einzelfasern (43) opake, Streulicht absorbierende Faserelemente vorgesehen sind.

5. Beleuchtungssystem (1) nach mindestens einem der vorhergehenden Ansprüche,
**dadurch gekennzeichnet, dass**
die Einzelfasern (43) der Faseranordnung Glasfasern bestehend aus einem Kern- und einem Mantelglas oder Quarzfasern aufweisen.

6. Beleuchtungssystem (1) nach Anspruch 5
**dadurch gekennzeichnet, dass**
die Einzelfasern (43) der Faseranordnung hohe Transmissionswerte im sichtbaren oder insbesondere im blauen sichtbaren Spektralbereich unterhalb 520 nm oder im nahen IR-Bereich bis 1000 nm aufweisen.

7. Beleuchtungssystem (1) nach einem der vorhergehenden Ansprüche,
**dadurch gekennzeichnet, dass**
das Kern- und Mantelglas, sowie die Abtrennung im Bereich C (46) aus einer Blei- bzw. Schwermetallfreien Glaszusammensetzung besteht.

8. Beleuchtungssystem (1) nach einem der Ansprüche 1 bis 4,
**dadurch gekennzeichnet, dass**
die Einzelfasern (43) aus optisch leitfähigen Kunststofffasern bestehen.

9. Beleuchtungssystem (1), nach einem der vorhergehenden Ansprüche,
**dadurch gekennzeichnet, dass**
der Faserstab (40) mindestens eine Biegung und/ oder eine Verjüngung zwischen dem proximalen und dem distalen Ende (41, 42) des Faserstabs (40) aufweist oder als räumlich verformter Faserstab (40) ausgeführt ist, der entlang seiner Längsachse einen unterschiedlichen Querschnitt aufweisen kann.

10. Beleuchtungssystem (1) nach einem der vorhergehenden Ansprüche,
**dadurch gekennzeichnet, dass**
das optische Element (50) am distalen Ende (42) als Gradientenindex-Linse (51), Kugellinse (52), asphärische Linse, Fresnel-Linse (53) oder als LSR-Linse ausgeführt ist.

11. Beleuchtungssystem (1) nach Anspruch 10 **dadurch gekennzeichnet, dass** das optische Element (50) in einer Abdeckscheibe (55) eingebettet und mit einem optischen Kleber (54) mit dem distalen Ende (42) des Faserstabs (40) verbunden oder in einer Bohrung (47) des Faserstabs (40) eingeklebt ist.

12. Beleuchtungssystem (1) nach einem der vorhergehenden Ansprüche, **dadurch gekennzeichnet, dass**
das Beleuchtungssystem an eine Lichtquelle anschließbar oder einer Lichtquelle zuordenbar ist oder an eine solche angeschlossen oder einer solchen zugeordnet ist, wobei die Lichtquelle ein optisches Koppelelement (20) umfasst, welches einen Strahlteiler (21) aufweist, in den im Betriebszustand das Licht der Lichtquelle (10) über ein Fokusierelement (13) in Form einer Linsenanordnung oder eines Reflektors einkoppelbar ist und das derart kollimierte Licht in das proximale Ende (41) des Faserstabs (40) einkoppelbar ist oder im Betriebszustand eingekoppelt wird, und dass das Bild am proximalen Ende (41) des Faserstabs (40) aus dem Bereich B (45) mit kleinerem Einzelfaserkerndurchmesser B (45.1) auf eine aktive Fläche (31) einer Kamera (30) oder eines Sensors mittels eines Linsensystems (22) projezierbar ist.

13. Beleuchtungssystem (1) nach Anspruch 12,
**dadurch gekennzeichnet, dass**
die Lichtquelle (10) zur Beleuchtung eines Objektes (60) mit seiner Objektoberfläche (61) einen LED-Typ A und zusätzlich einen LED-Typ B mit zum LED-Typ A unterschiedlicher Lichtfrequenz und das Einkoppelelement (20) an dessen Projektionsausgangs zur Kamera (30) einen optischen Filter (23) aufweist, der ein Durchlassverhalten für die Lichtfrequenz des LED-Typs B aufweist.

14. Beleuchtungssystem (1) nach Anspruch 13,
**dadurch gekennzeichnet, dass** die Lichtquelle (10) einen LED-Typ A mit einem Strahlungsspektrum von ≤ 520 nm und einen LED-Typ B mit einem Strahlungsspektrum im grünen, gelben oder roten sichtbaren Spektralbereich oder im IR-Spektralbereich aufweist.

15. Beleuchtungssystem (1) nach einem der vorhergehenden Ansprüche,
**dadurch gekennzeichnet, dass**
der Faserstab (40) mit seinen Bereichen A (44), B (45) und C (46) und dem optischen Element (50) am distalen Ende (42) einen optischen Abstandshalter (70) aufweist, wobei der optische Abstandshalter (70) als Hülse oder als transparenter Körper, bestehend aus Glas, Quarz oder Kunststoff.

16. Beleuchtungssystem (1) nach Anspruch 15, **dadurch gekennzeichnet, dass** der optische Abstandshalter (70) als als Lichtleitelement ausgeführt ist.

## Claims

1. Illumination system (1) for an inspection system, which is connected to a light source (10) and which comprises at least one spatial fibre arrangement formed from a multiplicity of individual fibres with an optical element (50) at the distal end (42) of the fibre arrangement, wherein the fibre arrangement has in its cross section at least one region B (45) having individual fibres (43) with a significantly smaller active diameter in comparison with the individual fibres (43) of a remaining region A (44) of the fibre arrangement, and wherein the optical element (50) at the distal end (42) is assigned at least to said region B (45),
wherein
the fibre arrangement is designed as a rigid fibre rod (40), wherein at least the individual fibres in the region A (44) and the individual fibres in the region B (45) are fused to one another or sintered together or adhesively bonded to one another at least in partial regions of their outer circumferential surfaces,
**characterized in that**
a region C (46) is provided between the region A (44) and the region B (45), said region C suppressing stray light transfers between the regions A and B (44, 45) of the fibre rod (40) in the operating state,
wherein the region C (46) is designed as an opaque or translucent or largely transparent or coloured separation,
the wall thickness C (46.1) of which, depending on the transmittance of the separation for the wavelength range of the light source (10), is dimensioned in such a way that stray light transfers between the regions A and B (44, 45) are damped by at least a factor of 10.

2. Illumination system (1) according to Claim 1, **characterized in that**
an optical coupling element (20) is provided at the proximal end (41) of the fibre rod (40), by means of which optical coupling element, in the operating state, light from the light source (10) can be coupled into the fibre rod (40) and an image at the proximal end (41) of the fibre rod (40) from the region B (45) can be coupled into a camera (30) or into a sensor.

3. Illumination system (1) according to at least one of the preceding claims,
**characterized in that**
an individual fibre core diameter B (45.1) in the region B (45) of the fibre rod (40) has a diameter of less than 25 µm.

4. Illumination system (1) according to at least one of the preceding claims,
**characterized in that**
opaque fibre elements that absorb stray light are provided between the individual fibres (43).

5. Illumination system (1) according to at least one of the preceding claims,
**characterized in that**
the individual fibres (43) of the fibre arrangement comprise optical fibres comprising a core glass and a cladding glass or quartz fibres.

6. Illumination system (1) according to Claim 5, **characterized in that**
the individual fibres (43) of the fibre arrangement have high transmission values in the visible or in particular in the blue visible spectral range below 520 nm or in the near IR range up to 1000 nm.

7. Illumination system (1) according to any of the preceding claims,
**characterized in that**
the core glass and cladding glass and also the separation in the region C (46) comprise a lead- or heavy-metal-free glass composition.

8. Illumination system (1) according to any of Claims 1 to 4,
**characterized in that**
the individual fibres (43) comprise optically conductive plastic fibres.

9. Illumination system (1) according to any of the preceding claims,
**characterized in that**
the fibre rod (40) has at least one bend and/or one taper between the proximal and distal ends (41, 42) of the fibre rod (40) or is designed as a spatially deformed fibre rod (40) which can have a varying cross section along its longitudinal axis.

10. Illumination system (1) according to any of the preceding claims,
**characterized in that**
the optical element (50) at the distal end (42) is designed as a gradient-index lens (51), a spherical lens (52), an aspherical lens, a Fresnel lens (53) or as an LSR lens.

11. Illumination system (1) according to Claim 10, **characterized in that** the optical element (50) is embedded in a cover sheet (55) and, by means of an optical adhesive (54), is connected to the distal end (42) of the fibre rod (40) or is adhesively bonded in a hole (47) of the fibre rod (40).

12. Illumination system (1) according to any of the preceding claims,
**characterized in that**
the illumination system is connectable to a light source or assignable to a light source or is connected to such a light source or is assigned to such a light source, wherein the light source comprises an optical coupling element (20) having a beam splitter (21), into which, in the operating state, the light from the light source (10) can be coupled via a focusing element (13) in the form of a lens arrangement or a reflector and the light collimated in this way can be coupled or in the operating state is coupled into the proximal end (41) of the fibre rod (40), and **in that** the image at the proximal end (41) of the fibre rod (40) from the region B (45) having a smaller individual fibre core diameter B (45.1) is projectable onto an active area (31) of a camera (30) or of a sensor by means of a lens system (22).

13. Illumination system (1) according to Claim 12, **characterized in that**
the light source (10) for illuminating an object (60) with the object surface (61) thereof comprises an LED type A and additionally an LED type B having a different light frequency than the LED type A, and the coupling-in element (20) comprises, at the projection output thereof to the camera (30), an optical filter (23) having a transmission response for the light frequency of the LED type B.

14. Illumination system (1) according to Claim 13, **characterized in that** the light source (10) comprises an LED type A having a radiation spectrum of ≤520 nm and an LED type B having a radiation spectrum in the green, yellow or red visible spectral range or in the IR spectral range.

15. Illumination system (1) according to any of the preceding claims,
**characterized in that**
the fibre rod (40) with its regions A (44), B (45) and C (46) and the optical element (50) comprises an optical spacer (70) at the distal end (42), wherein the optical spacer (70) is in the form of a sleeve or a transparent body, consisting of glass, quartz glass or plastic.

16. Illumination system (1) according to Claim 15, **characterized in that** the optical spacer (70) is designed as a light-guide element.

## Revendications

1. Système d'éclairage (1) qui est destiné à un système d'inspection, qui est relié à une source de lumière (10) et qui comprend au moins un ensemble de fibres spatial formé d'un grand nombre de fibres individuelles et muni d'un élément optique (50) à l'extrémité distale (42) de l'ensemble de fibres, l'ensemble de fibres comportant en coupe transversale au moins une zone B (45) qui comporte des fibres individuelles (43) ayant un diamètre actif nettement plus petit que celui des fibres individuelles (43) d'une zone restante A (44) de l'ensemble de fibres, et l'élément optique (50) étant associé, à l'extrémité distale (42), au moins à cette zone B (45),
l'ensemble de fibres étant conçu sous la forme d'une barre de fibres rigide (40),
au moins les fibres individuelles de la zone A (44) et les fibres individuelles de la zone B (45) étant fusionnées ou frittées ou collées ensemble au moins dans des sous-zones de leurs surfaces périphériques extérieures,
**caractérisé en ce que**
entre la zone A (44) et la zone B (45) est prévue une zone C (46) qui, dans l'état de fonctionnement, supprime le passage de lumière diffusée entre les zones A et B (44, 45) de la barre de fibres (40),
la zone C (46) étant conçue comme une séparation opaque ou translucide ou dans une large mesure transparente ou colorée dont l'épaisseur de paroi C (46.1) est dimensionnée, en fonction du degré de transmission de la séparation pour la gamme de longueurs d'onde de la source de lumière (10), de manière à atténuer d'au moins un facteur 10 des passages de lumière diffusée entre les zones A et B (44, 45).

2. Système d'éclairage (1) selon la revendication 1, **caractérisé en ce que**
un élément de couplage optique (20) est prévu à l'extrémité proximale (41) de la barre de fibres (40), lequel élément de couplage optique permet, dans l'état de fonctionnement, d'injecter par couplage de la lumière provenant de la source de lumière (10) dans la barre de fibres (40) et d'injecter par coulage une image, située à l'extrémité proximale (41) de la barre de fibres (40), depuis la zone B (45) jusque dans une caméra (30) ou dans un capteur.

3. Système d'éclairage (1) selon l'une au moins des revendications précédentes,
**caractérisé en ce que**
un diamètre de cœur de fibre individuelle B (45.1) dans la zone B (45) de la barre de fibres (40) a un diamètre inférieur à 25 µm.

4. Système d'éclairage (1) selon l'une au moins des revendications précédentes,
**caractérisé en ce que**
des éléments de fibre absorbant la lumière opaque et dispersée sont prévus entre les fibres individuelles (43) .

5. Système d'éclairage (1) selon l'une au moins des revendications précédentes,
**caractérisé en ce que**
les fibres individuelles (43) de l'ensemble de fibres comportent des fibres de verre comprenant une âme en verre et une gaine en verre des fibres de quartz.

6. Système d'éclairage (1) selon la revendication 5, **caractérisé en ce que**
les fibres individuelles (43) de l'ensemble de fibres ont des valeurs de transmission élevées dans le domaine spectral visible ou en particulier dans le domaine spectral visible bleu au-dessous de 520 nm ou dans le domaine proche IR jusqu'à 1000 nm.

7. Système d'éclairage (1) selon l'une des revendications précédentes,
**caractérisé en ce que**
le cœur en verre et la gaine en verre ainsi que la séparation dans la zone C (46) sont en une composition de verre sans plomb ou sans métaux lourds.

8. Système d'éclairage (1) selon l'une des revendications 1 à 4,
**caractérisé en ce que**
les fibres individuelles (43) comprennent des fibres en matière synthétique optiquement conductrices.

9. Système d'éclairage (1) selon l'une des revendications précédentes,
**caractérisé en ce que**
la barre de fibres (40) comporte au moins un coude et/ou un amincissement entre l'extrémité proximale et l'extrémité distale (41, 42) de la barre de fibres (40) ou est réalisée sous la forme d'une barre de fibres (40) spatialement déformée qui peut avoir une section transversale différente le long de son axe longitudinal.

10. Système d'éclairage (1) selon l'une des revendications précédentes,
**caractérisé en ce que**
l'élément optique (50) est réalisé, à l'extrémité distale (42), sous la forme d'une lentille à gradient d'indice (51), d'une lentille sphérique (52), d'une lentille asphérique, d'une lentille de Fresnel (53) ou d'une lentille LSR.

11. Système d'éclairage (1) selon la revendication 10, **caractérisé en ce que**
l'élément optique (50) est incorporé dans une plaque de recouvrement (55) et est relié à l'extrémité distale (42) de la barre de fibres (40) à l'aide d'un adhésif optique (54) ou est collé dans un alésage (47) de la barre de fibres (40).

12. Système d'éclairage (1) selon l'une des revendications précédentes,
**caractérisé en ce que**
le système d'éclairage peut être raccordé à une source de lumière ou peut être associé à une source de lumière ou est raccordé ou associé à une telle source de lumière, la source de lumière comprenant un élément de couplage optique (20) qui comporte un diviseur de faisceau (21) dans lequel la lumière de la source de lumière (10) peut être injectée par couplage, dans l'état de fonctionnement, par le biais d'un élément de focalisation (13) se présentant sous la forme d'un ensemble de lentilles ou d'un réflecteur et la lumière ainsi collimatée peut être injectée par couplage, ou est injectée par couplage dans l'état de fonctionnement, dans l'extrémité proximale (41) de la barre de fibres (40) et **en ce que** l'image, située à l'extrémité proximale (41) de la barre de fibres (40) de la zone B (45) ayant un diamètre de cœur de fibre individuel B plus petit (45.1), peut être projetée sur une surface active (31) d'une caméra (30) ou d'un capteur au moyen d'un système de lentilles (22).

13. Système d'éclairage (1) selon la revendication 12, **caractérisé en ce que**
la source de lumière (10) comporte une LED de type A et en plus une LED de type B, dont la fréquence de lumière est différente de celle de la LED de type A, afin d'éclairer un objet (60) pourvu de sa surface d'objet (61) et l'élément d'injection par couplage (20) comporte au niveau de sa sortie de projection vers la caméra (30) un filtre optique (23) qui a un comportement de transmission pour la fréquence de lumière de la LED de type B.

14. Système d'éclairage (1) selon la revendication 13, **caractérisé en ce que**
la source de lumière (10) comporte une LED de type A dont le spectre de rayonnement est ≤ 520 nm et une LED de type B dont le spectre de rayonnement est dans le domaine spectral visible vert, jaune ou rouge ou dans le domaine spectral IR.

15. Système d'éclairage (1) selon l'une des revendications précédentes,
**caractérisé en ce que**
la barre de fibres (40) pourvue de ses zones A (44), B (45) et C (46) et de l'élément optique (50) comporte un espaceur optique (70) à l'extrémité distale (42), l'espaceur optique (70) étant réalisé sous la forme d'un manchon ou d'un corps transparent en verre, en quartz ou en matière synthétique.

16. Système d'éclairage (1) selon la revendication 15, **caractérisé en ce que** l'espaceur optique (70) est réalisé sous la forme d'un élément formant guide de lumière.
